# EUROPEAN PATENT SPECIFICATION

(11) **EP 2 271 397 B1**
(45) Date of publication and mention of the grant of the patent: **13.02.2013**
(21) Application number: 09723940.4
(22) Date of filing: 27.03.2009
(51) Int. Cl.: A61M 39/00, A61M 39/10, F16L 37/24

(54) **CONNECTORS HAVING FEATURES TO FACILITATE OR HAMPER TIGHTENING AND/OR LOOSENING**
STECKVERBINDER MIT FUNKTIONEN ZUR ERLEICHTERUNG ODER ERSCHWERUNG VON DEREN ANZIEHUNG ODER LÖSUNG
CONNECTEURS AYANT DES ÉLÉMENTS FACILITANT OU GÊNANT LE SERRAGE ET/OU LE DESSERRAGE

(30) Priority: 06.02.2009 US 150659 P; 28.03.2008 US 40326 P
(43) Date of publication of application: 12.01.2011
(73) Proprietor: TecPharma Licensing AG, 3401 Burgdorf (CH)
(72) Inventor: SEIFERT, John, Coventry CT 06238 (US); LINDSAY, Mark, Ashford CT 06278 (US); WOODS, Jeffrey, Stafford Springs CT 06076 (US)
(86) International application number: PCT/US2009/038683
(87) International publication number: WO 2009/121029

(56) References cited:
- EP-A- 0 248 979
- WO-A-94/22520
- WO-A-97/36636
- WO-A-2008/014034
- GB-A- 632 317
- US-A- 5 557 070
- US-A- 5 947 954

## Description

### BACKGROUND

### Field of the Disclosure

The present disclosure relates generally to connectors, and more particularly to rotatable connectors.

### Description of the Related Art

Various connectors are used to connect medical devices. For example, connectors can be used in fluid delivery systems to connect syringes, catheters, lumens, tubes, or other devices. Such connectors generally aim to create a sealed environment between connectors and establish fluid communication between the connectors. It is generally desirable for such connectors to also be disconnectable, Among the connectors used in fluid delivery systems are luer-type connecters, which generally include frustoconical surfaces configured for mating engagement between male and female parts.
WO 2008/014034 discloses a luer fitting for a power injectable PICC. WO94/22520 discloses a luer connector which is provided with a tactile and audible torque indicator.

### SUMMARY

An aspect of at least one of the embodiments disclosed herein includes the realization that connectors may be over-tightened or under-tightened, and/or may be too easy or too difficult to disconnect. For example, a connector may be over-tightened by a user because of concern about creating a sealed environment in a fluid delivery system. Such occurrences may be particularly prevalent among diabetics who sometimes suffer from conditions that cause numbness or tingling in hands and/or fingers, which can render difficult the manipulation of small objects and slick surfaces.

Over-tightening may cause a connector to fracture or break upon connection or disconnection. Additionally or alternatively, an over-tightened connector may be difficult to loosen or disconnect. Conversely, under-tightening may result in leakage due to failure to create a seal between connectors, unintentional disconnection, or both.

It is an object of the present invention to provide a connector which avoids the mentioned problems, particularly diminishs the risk of over-tightening and increases the ease of loosening from another medical device.

The object is solved by a medical connector according to claim 1. Preferred embodiments are specified in the dependent claims.

Thus, in accordance with at least one of the embodiments disclosed herein, a connector can comprise a body and gripping feature which facilitates or hampers tightening and/or loosening.

In accordance with some examples, a connector can comprise a body and a gripping feature. The gripping feature can comprise a first end proximal to a central portion of the connector and a second end distal to the central portion of the connector. The gripping feature can further comprise a surface that extends in a curved path, such as a spiral, between the first end and the second end.

In accordance with some examples, a connector can comprise a body having a first cavity near a proximal end of the body, a second cavity near a distal end of the body, the first and second cavities forming a passageway through the entire length of the body, threads for connecting to at least one other device, at least one tapered surface for mating with another device, a central axis, and at least one gripping feature. The gripping feature can comprise a surface extending primarily radially near the central axis, and primarily circumferentially near the gripping feature's radially distant end.

In accordance with some examples, a connector can comprise a body having a first cavity near a proximal end of the body, a second cavity near a distal end of the body, the first and second cavities forming a passageway through at least a portion of the body. The connector can include threads for connecting to at least one other device, and/or at least one tapered surface for mating with another device. The connector can include a central axis, and at least one gripping feature. The gripping feature can extend primarily radially near the central axis, and primarily circumferentially near the gripping feature's radially distant end. In some embodiments, the gripping feature can be asymmetrical about any plane which contains the central axis.

In accordance with some examples, a gripping feature can comprise a flange with at least one radially outwardly facing surface. The flange can extend from the body of the connector and have a median line running through its cross-section. The flange can have a proximal portion, with the median line of the proximal portion extending primarily radially from the central axis of the connector body. The flange can also have a distal portion, with the median line of the distal portion extending generally at an angle from the median line of the proximal portion.

In accordance with some examples, a gripping feature can generally comprise a flange with at least one radially outwardly facing surface, the flange being asymmetrical about any plane containing the central axis of the connector body.

In accordance with some examples, a gripping feature can comprise a flange with at least one radially outwardly and at least one radially inwardly facing surface. The flange can protrude from the connector body at an angle such that its distal end or tip is close to the connector body than it is to the base of the flange.

In accordance with some examples, at least one gripping feature can comprise a first end proximal to a central portion of the body and a second end distal to the central portion of the body, the first end intersecting a first radial plane containing a central axis of the body, and the second end intersecting a second radial plane containing the central axis of the body, the first radial plane being different than the second radial plane such that the first and second ends are radially offset from one another.

In accordance with some examples, a connector can comprise a body and a gripping feature. The gripping feature can extend from the body, and can substantially flex and/or bend about the body so as to generally collapse around the body.

In accordance with yet another example, a connector can comprise a body, a gripping feature, and a stop. The gripping feature can extend from the body, and can flex about the body when pressure is applied to the gripping feature. The stop can have at least one surface which inhibits the gripping feature from flexing in at least one direction.

In accordance with yet another example, a connector can comprise a body and a gripping feature. The gripping feature can include at least one slot and/or opening, such that the connector has a generally uniform thickness of material throughout.

### BRIEF DESCRIPTION OF THE DRAWINGS

The above-mentioned and other features, aspects, and advantages of the inventions disclosed herein are described below with reference to drawings of one embodiment. The illustrated embodiment is intended to illustrate, but not limit, the inventions. The drawings contain the following figures:

Figure 1 is a perspective view of an embodiment of a connector.

Figure 2 is a side plan view of the embodiment of Figure 1.

Figure 3 is a cross-sectional view of the embodiment of Figure 1 along line III - III, shown in Figure 2.

Figure 4 is a cross-sectional view of the embodiment of Figure 1 along line IV - IV, shown in Figure 2.

Figure 5A is a cross-sectional view of an embodiment of a connector.

Figure 5B is a cross-sectional view of an embodiment of a connector.

Figure 5C is a cross-sectional view of an embodiment of a connector.

Figure 5D is a cross-sectional view of the embodiment of Figure 5C, with the gripping features in a flexed configuration.

Figure 6A is a cross-sectional view of an embodiment of a connector.

Figure 6B is a cross-sectional view of the embodiment of Figure 6A, with the gripping features in a flexed configuration.

Figure 6C is a cross-sectional view of an embodiment of a connector.

Figure 7 is a cross-sectional view of an embodiment of a connector.

### DETAILED DESCRIPTION OF THE PREFERRED EMBODIMENTS

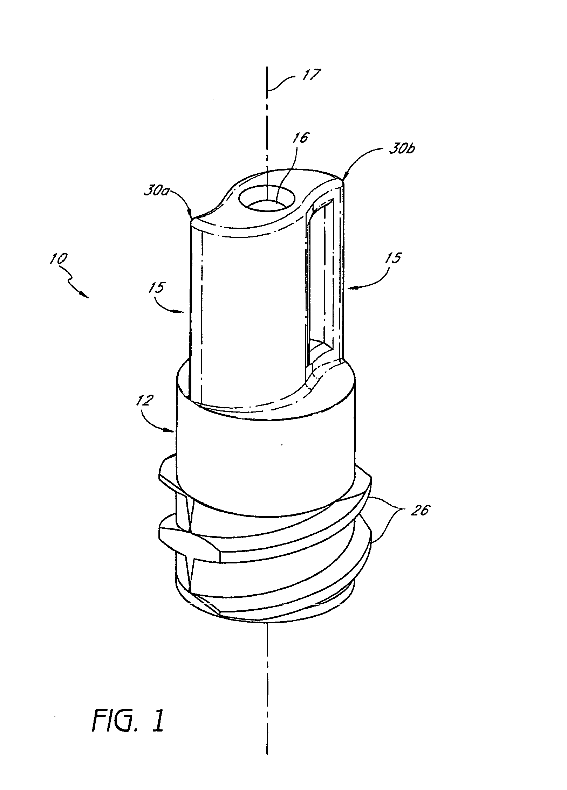

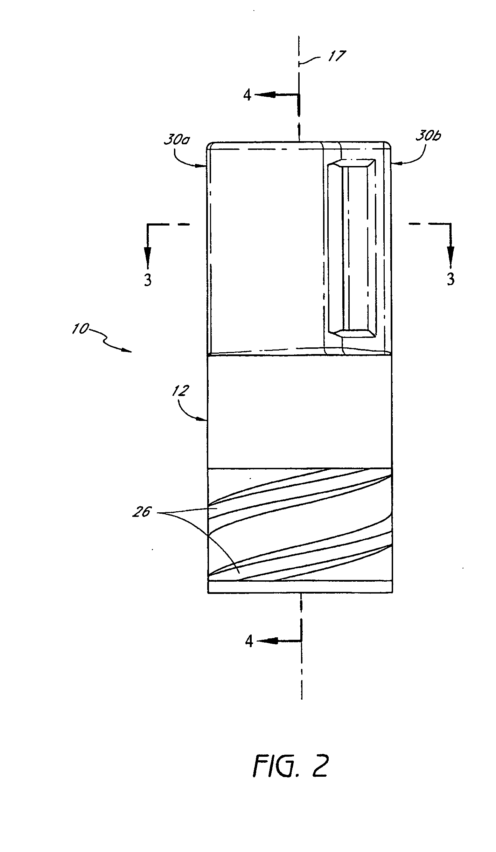

Figures 1-4 illustrate an embodiment of a connector 10. The illustrated connector 10 is a luer-type connector. However, other embodiments may comprise other types of connectors. The connectors can be formed from plastic, preferably polyethylene terephthalate (PET), polypropylene, or other types of plastic suitable for injection molding. For example, in at least one embodiment the connectors can be formed from polyvinyl chloride (PVC). Other materials can also be used, including but not limited to metal.

In the illustrated embodiment of Figures 1-4, the connector 10 comprises a body 12, a passageway 11, a fastener 13, and features 15 for gripping and rotating the connector to tighten or loosen the connector.

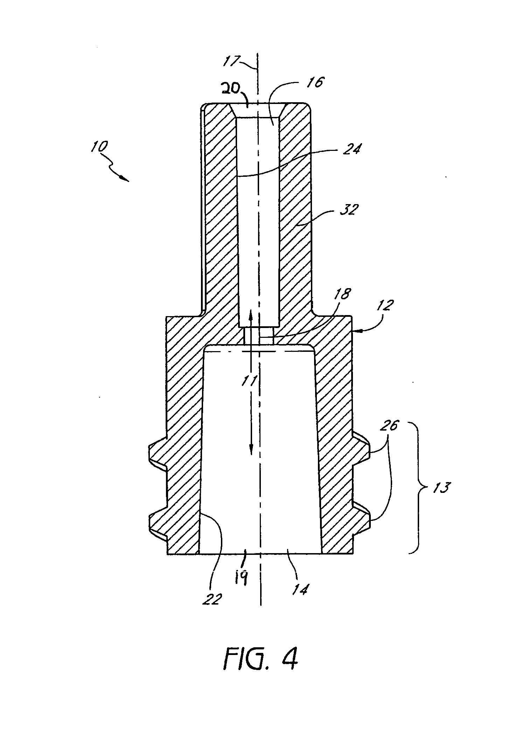

The passageway 11 can comprise one or more cavities. For example, the passageway 11 can comprise a first cavity 14 and a second cavity 16 that are disposed in proximity to opposite ends of the body, as shown in Figure 4. In some embodiments, the first cavity 14 can comprise an opening 19 near or at an end of the body 12. Additionally or alternatively, the second cavity 16 can comprise an opening 20 near or at an end of the body 12. In some embodiments, the opening 19 of the first cavity 14 and the opening 20 of the second cavity 16 can be positioned near or at opposing ends of the body 12. In some embodiments, the connector can comprise more or fewer than two cavities. For example, the connector may comprise a single cavity, three cavities, or no cavity. In at least one embodiment, the connector 10 can comprise a plug, and/or the connector 10 can comprise a cap. The one or more cavities, if present, can permit fluid to flow through the connector 10.

The first cavity 14 of the illustrated embodiment has a slightly tapered surface 22. The tapered surface 22 can be sized and shaped to substantially mate with one or more complementary surfaces of another connector. In at least one embodiment, the connector 10 can conform to the ISO 594 standard for luer tapers. In yet other embodiments the connector 10 can have non-standard tapers. In at least one embodiment, the first cavity 14 can have geometries different than the geometry shown in the illustrated embodiment.

The second cavity 16 can be sized and shaped to receive a tube (not shown). The second cavity 16 can have a tapered surface 24 that facilitates bonding to the tube when the tube is inserted into the cavity 16. The second cavity 16 may have geometries different than the geometry shown in the illustrated embodiment.

The first cavity 14 and the second cavity 16 can be separated by a neck 18, as illustrated in Figure 4. The neck 18 can serve one or more purposes. For example, the neck 18 can limit insertion of a tube into the cavity 16. Additionally or alternatively, the neck 18 may permit fluid to flow through the passageway 11 between the first cavity 14 and the second cavity 16. For example, in some embodiments the passageway 11 comprises an opening through which fluid flows from the first cavity 14 to the second cavity 16. In some embodiments, the opening extends through the entire length of the connector 10.

The flow rate of the fluid moving through passageway 11 can be related to the cross-sectional area of the passageway. The selection and design of the size and shape of the cross-section of the passageway 11 can affect the flow rate of fluid moving between the first and second cavities 14, 16. Thus, various sizes, shapes, and configurations of the cross-section, including but not limited to that shown in the illustrated embodiment, can be used.

The fastener 13 can attach the connector 10 to a fluid transfer device, such as a syringe or tube, having a complementary connector. With reference to Figure 4, the fastener 13 of the connector 10 comprises partial threads 26. Alternatively, the fastener can comprise full threads, tabs, rings, or other structures capable of fastening the connector to a complementary connector. The threads can be single threads, double threads, or any other thread combination. As illustrated in Figure 4, the threads 26 can be located on the exterior surface of the body 12. In some embodiments, the threads can be on an interior surface or surfaces of body 12.

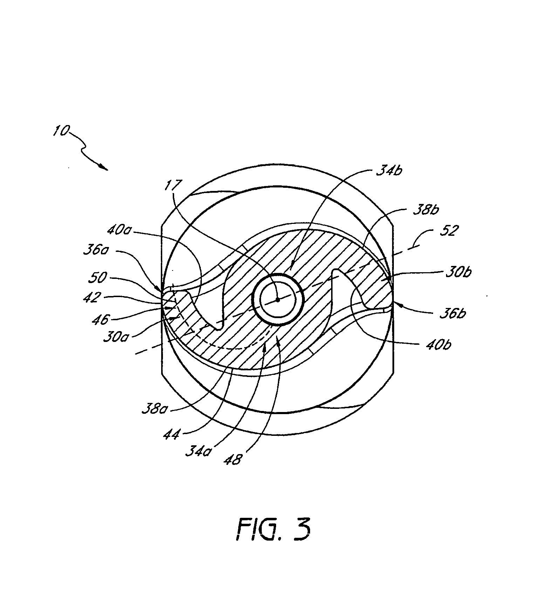

In the embodiment illustrated in Figures 1-4, the threads 26 are configured such that a connection between the connector 10 and a complementary connector is tightened as the connector 10 is rotated relative to the complementary connector in a clockwise direction from the perspective of Figure 3. In other embodiments, the connector 10 can comprise threads, or other fastener 13, that are configured such that a connection between the connector 10 and a complementary connector is tightened as the connector 10 is rotated relative to the complementary connector in a counter-clockwise direction from the perspective of Figure 3. Nevertheless, if the connector 10 is not appropriately tightened to a complementary connector, the connection may leak or one or both of the connectors may break.

The gripping features 15 can facilitate manipulation, including rotation, of the connector 10. In various embodiments, the gripping features can comprise flanges, wings, protruding segments, recesses, and/or indentations located on or within the body of the connector. The gripping features can have curved and/or angled surfaces that can be engaged by fingers.

In the illustrated embodiment, the connector 10 comprises two of the gripping features 15. While the present embodiment has two gripping features, other embodiments can have only a single gripping feature or can have more than two gripping features. The gripping features 15 can be located near a central portion 32 of the body 12, which may surround the second cavity 16. In other embodiments, gripping features can be spaced from a central portion of the body.

The gripping features 15 can be elongate members that extend generally parallel to an axis of the connector, such as central axis 17, shown in Figures 1-3. In some embodiments, gripping features 15 can extend along approximately half of a length of the connector 10, while in other embodiments the gripping features can extend along more or less than half of the length of the connector. In the embodiment shown in Figures 1-4, the gripping features 15 extend along the majority of a length of the second cavity 16. However, in other embodiments the gripping features 15 can extend along all or a portion of a different portion of the body 12, such as the first cavity 14. Additionally, the gripping features can be of various sizes. For example, in some embodiments the outermost portions of the gripping features 15 can extend farther radially from a central axis 17 than does the body 12. With reference to Figure 3, each gripping feature 15 can comprise a first end 34a, 34b that is proximal to the central portion 32 of the body 12 and a second end 36a, 36b that is distal to the central portion 32. Each gripping feature 15 generally comprises at least a first surface 38a, 38b and a second surface 40a, 40b. The first surface 38a, 38b and the second surface 40a, 40b can be arranged to be generally opposing.

The surfaces 38a and 38b can extend between the first ends 34a, 34b and the second ends 36a, 36b. The surfaces 38a, 38b can comprise one or more segments that are curved or generally flat. In some embodiments, the surfaces 38a, 38b can face radially outwardly, as illustrated in Figure 3. The surfaces 38a, 38b can have at least a portion, or a group of portions, that is generally convex.

In some embodiments, each surface 38a, 38b of the gripping features 15 can have a distal segment 42 that is near the second end 36a, 36b, and a proximal segment 44 that is nearer to the first end 34a, 34b than is the distal segment 42. The surface area of the ends 36a, 36b can be narrow or substantially smaller than the surface area of the lateral surfaces 38a, 38b, such that a user's fingers would be less comfortable and/or more likely to slide off when gripping the ends than the lateral surfaces 38a, 38b. The distal segment 42 can extend in a direction that is generally circumferential about a central axis, while the proximal portion 44 can extend in a direction that has a greater radial component with respect to the central axis than does the direction of the distal portion 42. In some embodiments, as shown in Figure 3, the distal segment 42 can extend primarily circumferentially around the central axis 17. In some embodiments, the proximal portion 44 can extend primarily radially from a center of the connector 10. In some embodiments, the proximal portion 44 can be near the first ends 34a, 34b.

In some embodiments, the surfaces 38a, 38b can extend in a generally spiral path between the first ends 34a, 34b and second ends 36a, 36b, as shown in Figure 3. In some embodiments, as shown in Figures 1-4, the distal segment 42 can comprise the second end 36a, 36b of the gripping feature 15, and the second ends 36a, 36b can be substantially the farthest points measured radially from a central axis of the connector. In some embodiments, the gripping feature 15 does not have any portion that extends substantially farther from a central axis of the connector than does the distal segment 42. In some embodiments, the surface 38a, 38b can be generally convex and/or does not have any portion that is concave and located farther from a central axis of the connector than is the distal segment 42.

With reference to Figure 3, the surfaces 40a and 40b of the gripping features 15 can extend between the first ends 34a, 34b and the second ends 36a, 36b. The surfaces 40a and 40b can comprise one or more segments that are curved or generally flat. The surfaces 40a, 40b can have at least a portion, or a group of portions, that is generally concave. In some embodiments, the surfaces 40a, 40b can face radially inwardly as illustrated in Figure 3. In other embodiments, the surfaces 40a, 40b can face radially outwardly. In some embodiments, such as the embodiment of Figure 3, the gripping feature 15 can comprise a flange 30a, 30b having a distal portion 46, a proximal portion 48, and a median line 50 extending between the first surface 38a, 38b and the second surface 40a, 40b at a location equidistant between them. The proximal portion 48 can be located near the central axis of the connector 17. The distal portion 46 can be located farther from the central axis 17 than the proximal portion 48. Through the proximal portion 48, the median line 50 can extend primarily radially from the central axis 17. Through the distal portion 46, the median line 50 can extend in a direction that is more circumferential than the direction of that portion of the median line which passes through the proximal portion 48.

In some embodiments, the gripping feature 15 can comprise a flange that is asymmetrical about any plane containing a central axis of the connector body. For example, as shown in Figure 3, the flanges 30a, 30b are asymmetrical about a plane 52 and any other plane that contains the central axis 17. In some embodiments, the gripping feature 15 can comprise a first end 34a proximal to a central portion 32 of the body 12 and a second end 36a distal to the central portion 32 of the body 12, the first end 34a intersecting a first radial plane containing the central axis 17, and the second end 36a intersecting a second radial plane containing the central axis 17, the first radial plane being different than the second radial plane such that the first and second ends are radially offset from one another. In some embodiments the first radial plane intersects a portion of the first end 34a. For example, in some embodiments the first radial plane intersects a center portion of the first end 34a. In some embodiments, the second radial plane intersects a portion of the second end 36a. For example, in some embodiments the second radial plane intersects a center portion of the second end 36a.

Although in some embodiments the gripping feature or features 15 can comprise flanges, as indicated above, in other embodiments the gripping features 15 can have other configurations. For example, the gripping features 15 can comprise protrusions, wings, recesses, pockets, or grooves within at least a portion of the connector body. In some embodiments, a gripping feature 15 can comprise a protrusion that has a wave-like configuration with a thick base near a central axis of the connector body and a narrower tip. To connect or disconnect the connector 10, a user's fingers apply forces or pressure to the gripping features 15. The applied pressure can be considered to have a circumferential component, a normal component, and a tangential component. The circumferential component creates torque that turns the connector. The normal component contributes to friction that allows a user to maintain a grip on the connector while it is being manipulated, including turning. The tangential component, which acts in a direction along the contacted surface, can cause the user's fingers to slip relative to the contacted surface if they exceed the frictional force resulting from the normal component.

The torque required to turn the connector generally increases as the connector is tightened. Thus, the circumferential component of the applied pressure would need to be increased as the connector is tightened. A configuration of the connector in which pressure is applied against the surfaces 38a, 38b to tighten the connector, such as in the manner shown in Figures 1-4, can hamper a user's ability to tighten the connector. To tighten such a connector, a user can apply a pressure that has both a circumferential component that is sufficient to rotate the connector and a normal component that is sufficient to maintain the user's grip on the connector. Yet, a user's ability to apply pressure to the surfaces is limited. The configuration of surfaces 38a, 38b results in there being some required torque at which the maximum pressure applied by the user does not have both a normal component sufficient to maintain a grip and a circumferential component sufficient to overcome the required torque. In such a circumstance, application of a circumferential component that is sufficient to overcome the required torque also causes the tangential component to exceed the frictional force, which results in the user's fingers slipping off. Thus, the configuration of the surfaces 38a, 38b can reduce the incidence of over-tightening and the risk of damaging one or both connectors.

On the other hand, a configuration of the connector in which pressure is applied to some portion of the surfaces 40a, 40b, or to an area adjacent the surfaces 40a, 40b, to tighten the connector can facilitate a user's ability to tighten the connector. The configuration of the surfaces 40a, 40b tends to retain a user's finger in contact with the connector. For example, when a connector is being tightened by applying pressure to the surfaces 40a, 40b, the normal component required to maintain a grip on the connector generally will not increase as the connector is tightened. Instead, the gripping feature can provide support for the fingers. Thus, a greater component of the applied pressure can act in a circumferential direction to turn the connector, thereby facilitating tightening of the connector and reducing the occurrence of leakage.

In contrast to tightening, the torque required to rotate the connector generally decreases as a connector is loosened. Thus, the circumferential component of the applied force may be lessened as the connector is loosened. Nonetheless, the connectors cannot be rotated to loosen the connection until static friction is overcome.

A configuration of the connector in which pressure is applied against the surfaces 40a, 40b, or to an area adjacent the surfaces 40a, 40b, to loosen the connector, such as in the manner shown in Figures 1-4, can facilitate a user's ability to loosen the connector. As noted above, the configuration of the surfaces 40a, 40b tends to retain a user's finger in contact with the connector. Thus, a greater component of the applied pressure can act in a circumferential direction to turn the connector, thereby facilitating loosening of the connector.

On the other hand, a configuration of the connector in which pressure is applied against the surfaces 38a, 38b to loosen the connector, can hamper a user's ability to loosen the connector. As noted above, the configuration of the surfaces 38a, 38b limits a user's ability to rotate the connector against a required torque. Thus, the configuration of surfaces 38a, 38b can hamper the loosening of a connector.

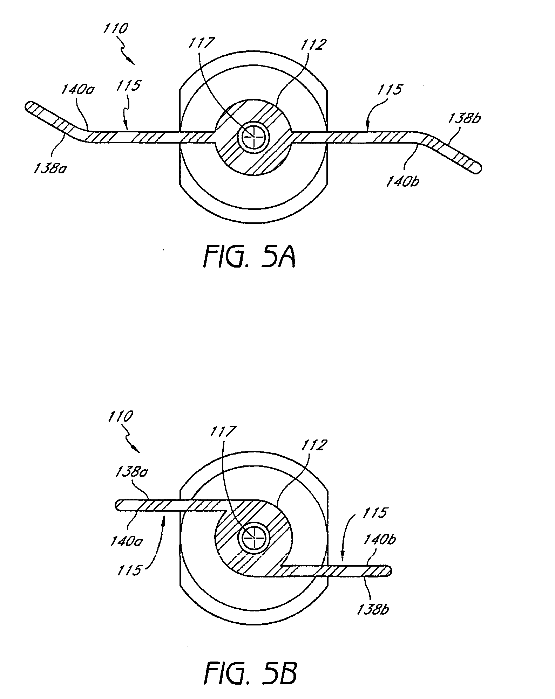

Figures 5A 5B, 5C, and 5D illustrate further examples of a connector 110. The connector 110 can be similar to the connector 10. Therefore, similar components of the connector 110 are referenced by the same reference numeral as the corresponding component, in the connector 10 incremented by one hundred. As with the connector 10, the connector 110 can comprise a body 112, central axis 117, and features 115 for gripping and rotating the connector to tighten or loosen the connector.

Like the gripping features 15, the gripping features 115 can facilitate manipulation, including rotation, of the connector 110. In the shown examples, the gripping features can comprise flanges, wings, and/or protruding segments connected to the body 112 of the connector, either directly or indirectly. In some examples the gripping features can be integrally formed with the body 112.

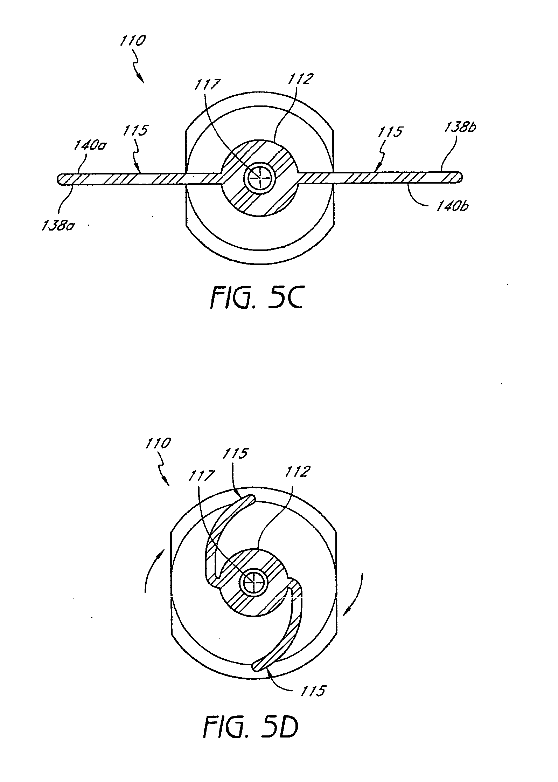

The gripping features 115 can differ from the gripping features 15 shown in Figure 1, in some respects. For example, and with reference to Figure 1, portions of the gripping features 15 nearest the partial threads 26 along axis 17 can generally be restrained from flexing relative to body 12 and axis 17 at least in part by their connection with the body 12 in this area. In contrast, the gripping features 115 shown in Figures 5A, 5B, 5C, and 5D can freely flex relative to body 112 and axis 117. For example, the gripping features 115 can be flexed or twisted such that they collapse around the body 112 as shown in Figure 5D (arrows indicating direction of movement). In some examples, the gripping features 115 can collapse against the body 112.

The gripping features 115 can have various cross-sectional profiles when unflexed. For example, the gripping features 115 can initially have a curved cross-sectional profile, as illustrated in Figure 5A. With reference to Figures 5B and 5C, the gripping features 115 can alternatively have a generally straight cross-sectional profile. Other configurations are also possible. As described below, the ability of the gripping features 115 to flex and/or bend, as shown in Figure 5D, can facilitate or hamper the tightening and/or loosening of the connector 110.

The torque required to turn the connector 110 can generally increase as the connector is tightened. Thus, the circumferential component of a pressure applied to the gripping features 115 would need to be increased as the connector is tightened. To tighten the connector 110, a user can apply a pressure that has both a circumferential component that is sufficient to rotate the connector and a normal component that is sufficient to maintain the user's grip on the connector. Yet, a user's ability to apply pressure to the surfaces of the gripping features 115 to turn the connector can be limited. For example, there can be some required torque at which the maximum pressure applied by the user does not have both a normal component sufficient to maintain a grip and a circumferential component sufficient to overcome the required torque. In such a circumstance, application of a circumferential component that is sufficient to overcome the required torque also causes the tangential component to exceed the frictional force, which results in the user's fingers slipping off.

With reference to Figures 5C and 5D, as pressure is applied to the surfaces 138a, 138b to turn the connector 110, the gripping features 115 can flex and/or bend in the direction the connector is being turned (see arrows in Figure 5D). As the gripping features flex, the amount of force required to maintain a grip on the connector can increase, and the tangential force being applied by the user to turn the connector 110 can result in the user's fingers slipping off. Thus, a user's ability to apply pressure to the surfaces of the gripping features 115 can be limited by the ability of the gripping features 115 to flex and/or bend. Where the user applies pressure to the surfaces 138a, 138b as shown in Figure 5C to tighten the connector, the ability of the gripping features 115 to flex and/or bend can thus reduce the incidence of over-tightening and the risk of damaging the connector.

In a further example, the ability of the gripping features 115 to flex and/or bend can be greater in one direction than in another. With reference to Figure 5A, in an example the gripping features 115 can have a bent profile at their ends. As pressure is applied to the surfaces 140a, 140b, the bent ends of the gripping features 115 can at least initially straighten as the gripping features 115 bend or flex. Thus, the gripping features 115 can bend more quickly and easily when pressure is applied to surfaces 138a and 138b, as opposed to surfaces 140a and 140b. When the connector shown in Figure 5A is being turned by applying pressure to the surfaces 140a, 140b, the gripping features 115 may only flex slightly, or not at all, and the normal component required to maintain a grip on the connector as the connector is turned can generally remain lower than if pressure is applied to surfaces 138a, 138b. Thus, a greater component of the applied pressure can act in a circumferential direction to turn the connector, resulting in increased support for the fingers and facilitating turning of the connector 110. Where the user applies pressure to the surfaces 140a, 140b as shown in Figure 5A to tighten the connector, tightening can be facilitated and the occurrence of leakage can be reduced.

With continued reference to Figure 5A, a configuration of the connector 110 in which pressure is applied against the generally concave surfaces 140a, 140b, or to an area adjacent the surfaces 140a, 140b, can facilitate a user's ability to loosen the connector. As noted above, the bent or angled profile of the gripping features 115 shown in Figure 5A can tend to retain a user's finger in contact with the connector 110. Thus, a greater component of the applied pressure can act in a circumferential direction to turn the connector, thereby facilitating loosening of the connector. On the other hand, a configuration of the connector 110 in which pressure is applied against the generally convex surfaces 138a, 138b to loosen the connector, can hamper a user's ability to loosen the connector, since applying pressure to surfaces 138a, 138b causes the gripping features 115 to flex relative to the body 112.

With reference to the example shown in Figure 5B, the gripping features 115 can be offset from the central axis 117. For example, the gripping features 115 can bend more quickly and easily when pressure is applied to surfaces 138a and 138b, as opposed to surfaces 140a and 140b. When the connector shown in Figure 5B is being turned by applying pressure to the surfaces 140a, 140b, the gripping features 115 may only flex slightly, or not at all, and the normal component required to maintain a grip on the connector as the connector is turned can generally remain lower than if pressure is applied to surfaces 138a, 138b. Thus, a greater component of the applied pressure can act in a circumferential direction to turn the connector, resulting in increased support for the fingers and facilitating turning of the connector 110. Where the user applies pressure to the surfaces 140a, 140b as shown in Figure 5B to tighten the connector, tightening can be facilitated and the occurrence of leakage can be reduced.

With continued reference to Figure 5B, a configuration of the connector 110 in which pressure is applied against the surfaces 140a, 140b, or to an area adjacent the surfaces 140a, 140b, can facilitate a user's ability to loosen the connector. As noted above, the offset nature of the gripping features 115 shown in Figure 5B can tend to retain a user's finger in contact with the connector 110. Thus, a greater component of the applied pressure can act in a circumferential direction to turn the connector, thereby facilitating loosening of the connector. On the other hand, a configuration of the connector 110 in which pressure is applied against the surfaces 138a, 138b to loosen the connector can hamper a user's ability to loosen the connector, since applying pressure to surfaces 138a, 138b causes the gripping features 115 to flex relative to the body 112.

Alternatively, the gapping features 115 illustrated in Figure 5B can be rigid, and substantially resist flexure. For example, the configuration of surfaces 138a, 138b in Figure 5B results in there being some required torque at which the maximum pressure applied by the user does not have both a normal component sufficient to maintain a grip and a circumferential component sufficient to overcome the required torque. In such a circumstance, application of a circumferential component that is sufficient to overcome the required torque also causes the tangential component to exceed the frictional force, which is more likely to result in the user's fingers slipping off. Thus, the configuration of the surfaces 138a, 138b in Figure 5B can reduce the incidence of over-tightening and the risk of damaging one or both connectors.

A configuration of the connector 110 in which pressure is applied to some portion of the surfaces 140a, 140b shown in Figure 5B, or to an area adjacent the surfaces 140a, 140b, to tighten the connector can facilitate a user's ability to tighten the connector. The configuration of the surfaces 140a, 140b tends to retain a user's finger in contact with the connector. For example, when a connector is being tightened by applying pressure to the surfaces 140a, 140b, the normal component required to maintain a grip on the connector generally will not increase as the connector is tightened. Instead, the gripping feature can provide support for the fingers. Thus, a greater component of the applied pressure can act in a circumferential direction to turn the connector, thereby facilitating tightening of the connector and reducing the occurrence of leakage.

Similarly, a configuration of the connector 110 shown in Figure 5B in which pressure is applied against the surfaces 140a, 140b, or to an area adjacent the surfaces 40a, 40b, to loosen the connector, can facilitate a user's ability to loosen the connector. On the other hand, a configuration of the connector 110 shown in Figure 5B in which pressure is applied against the surfaces 138a,138b to loosen the connector, can hamper a user's ability to loosen the connector.

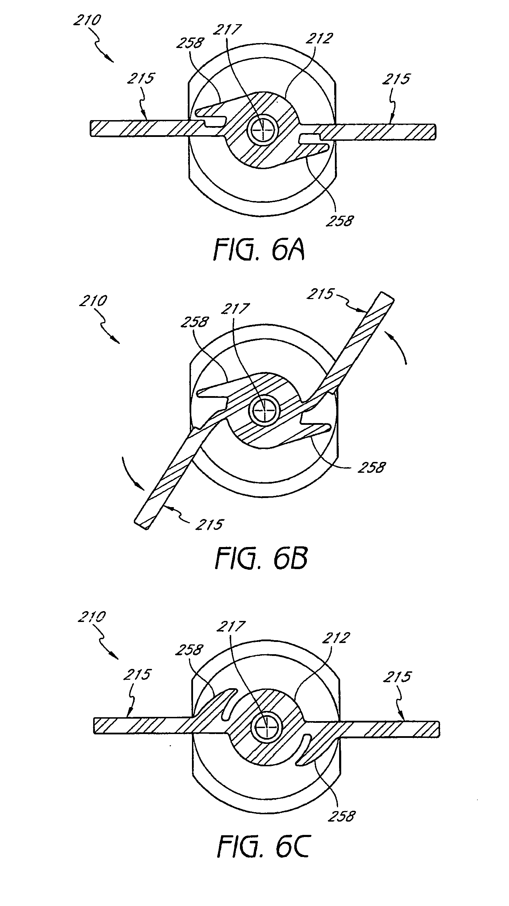

Figures 6A, 6B, and 6C illustrate examples of a connector 210. The connector 210 can be similar in many respects to the connectors 10 and 110. Therefore, similar components of the connector 210 are referenced by the same reference numeral as the corresponding component in the connector 10 incremented by two hundred. As with the connector 10, the connector 210 can comprise a body 212, a central axis 217, and features 215 for gripping and rotating the connector to tighten and/or loosen the connector.

With reference to Figures 6A, 6B, and 6C, the gripping features 215 can extend from a portion of the body 212. The gripping features 215 can have various configurations, including but not limited to those illustrated in Figures 6A, 6B, and 6C.

The connector 210 can include stops 258. For example, the stops 258 can be formed integrally with the body 212, as illustrated in Figures 6A and 6B. The stops can comprise at least one portion protruding generally radially away from the body 212 and alongside a portion of the gripping features 215. The stops 258 can inhibit or prevent the gripping features 215 from flexing and/or bending in at least one direction. For example, as the gripping feature 215 flexes in a counterclockwise direction (see arrow in Figure 6B), the gripping features 215 can freely flex without being inhibited by one of the stops. In contrast, as the gripping feature 215 is flexed in a clockwise direction, the gripping feature 215 can encounter stop 258, and be inhibited from further flexion.

Configurations and geometries for the stops 258 other than those illustrated in Figures 6A and 6B are also possible. For example, the stops 258 can be incorporated on, or form a part of, the gripping features 215 themselves, as illustrated in Figure 6C. Thus, as illustrated in Figure 6C, contact between the stops 258 and the body 212 can prevent further flexing of the gripping features 215 in one or more directions, rather than contact between the gripping features 215 and the stops 258 restricting flexion.

In examples, wherein the gripping features 215 are able to flex and/or bend, overtightening and/or loosening can be hampered, as with the connector 110. By incorporating stops 258, the flexing and/or beading can be controlled such that the gripping features 215 are able to flex and/or bend when the connector 210 is turned in one direction, but are generally inhibited or prevented from flexing and/or bending when turned in another direction.

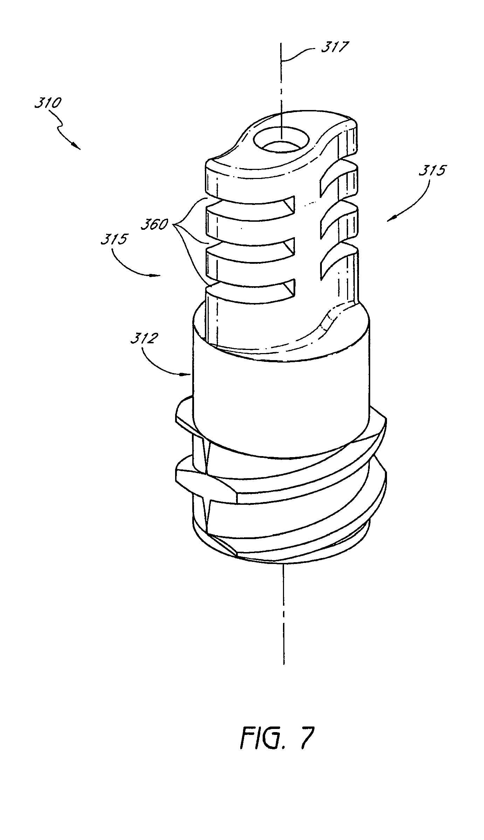

Figure 7 illustrates an embodiment of a connector 310. The connector 310 is similar in many respects to the connectors 10, 110, and 210. Therefore, similar components of the connector 310 are referenced by the same reference numeral as the corresponding component in the connector 10 incremented by three hundred. As with the connector 10, the connector 310 can comprise a body 312, a central axis 317, and features 315 for gripping and rotating the connector to tighten and/or loosen the connector.

With reference to Figure 7. the connector 310 can include at least one slot 360. In some embodiments, the slot 360 can extend entirely through a portion of the connector 310. In some embodiments, the connector 310 can include a series of slots 360 extending through a portion or portions of the gripping features 315. The slots 360 can aid In the manufacturing process by ensuring relatively uniform thickness of material throughout the connector 310, preventing warping, and facilitating accurate molding. Relatively uniform thicknesses of material can aid in the injection molding process by preventing unwanted warping of the connector material.

In some embodiments, the gripping features 15, 115, 215, and 315 described above can be comprised of different material than the body of the connector, and/or be separate components. For example, in some embodiments the gripping features 115 can be comprised of a more flexible and/or soft material than that of the body of the connector.

Of course, it is to be understood that not necessarily all objectives or advantages described may be achieved In accordance with any particular embodiment described herein. Some embodiments can include one or more features described in connection with one or more of the embodiments described herein. For example, the stops 258 shown in Figures 6A-6C can be used with the connector 10 shown in Figures 1-4, and with the connectors 110 shown in Figures 5A-5D. The slot 360 shown in Figure 7 can be used with the connectors 10 shown in Figures 1-4, with the connectors 110 shown In Figures 5A-5D, and with the connectors 210 shown in Figures 6A-6C. Additionally, in some embodiments a connector can include one or more different types of gripping features. For example, the connector can include one or more of the gripping features shown in Figures 1-4, plus one or more gripping features shown in Figures 5A-D, 6A-C, and Figure 7. Accordingly, the invention is not intended to be limited by the specific disclosures of preferred embodiments herein.

## Claims

1. A rotatable medical connector (10) comprising:
a body (12);
a central axis (17) extending through the body;
at least one gripping feature (15) extending from the body, the at least one gripping feature comprising a first substantially convex surface (38a) and a second substantially concave surface (40a) on a generally opposing side thereof, the at least one gripping feature further comprising a first end (34a), a second end (36a), a distal segment (42) located near the second end, and a proximal segment (44) located nearer to the first end than the distal segment, the proximal segment extending in a direction that has a greater radial component with respect to the central axis than does the direction of the distal segment, the distal segment extending in a direction that is generally circumferential about the central axis;
wherein the substantially convex surface provides substantially greater finger contact area than the substantially concave surface, such that the connector is configured to diminish the risk of over-tightening and increase the ease of loosening the connector from another medical device.

2. The rotatable connector of Claim 1, further comprising:
a first cavity (14) near a proximal end of the body, and a second cavity (16) near a distal end of the body, the first and second cavities forming a passageway (11) through at least a portion of a length of the body;
wherein the gripping feature extends asymmetrically about any plane containing the central axis.

3. The rotatable connector of Claims 1 or 2, wherein the gripping feature comprises a proximal portion (48) located near the central axis, with a median line (50) of the proximal portion extending primarily radially from the central axis, and a distal portion (46) located farther from the central axis than the proximal portion, with a median line (50) of the distal portion extending generally at an angle from the median line of the proximal portion.

4. The rotatable connector of any preceding claim, wherein the gripping feature comprises a flange (30a) with at least one radially outwardly facing surface, the flange being asymmetrical about any plane containing the central axis.

5. The rotatable connector of any preceding claim, wherein the gripping feature comprises a flange with at least one radially outwardly and at least one radially inwardly facing surface.

6. The rotatable connector of any preceding claim, wherein the gripping features is generally free to substantially flex relative to the body and central axis.

7. The rotatable connector of any preceding claim, wherein the gripping feature is generally inhibited from flexing in one direction and free to move in another, opposite direction.

8. The rotatable connector of any preceding claim, wherein the gripping feature is substantially flexed about the body when pressure is applied to at least one surface of the gripping feature.

9. The rotatable connector of any preceding claim, wherein the gripping feature is configured to substantially collapse about the body when pressure is applied to at least one surface of the gripping feature.

10. The rotatable connector of any preceding claim, further comprising threads (26) for connecting the connector device to at least one other device.

11. The rotatable connector of any preceding claim, further comprising at least one tapered surface for mating the connector device with a device.

12. The rotatable connector of any preceding claim, wherein the gripping feature extends axially along a portion of the body of the connector.

13. The rotatable connector of any preceding claim, further comprising at least one additional gripping feature extending from the body.

14. The rotatable connector of any preceding claim, wherein the gripping feature is comprised of different material than the body.

15. The rotatable connector of any preceding claim, wherein the gripping feature has a generally curved cross-sectional profile.

16. The rotatable connector of Claim 1, wherein the at least one gripping feature comprises surfaces that extend in a generally spiral path about the central axis.

## Patentansprüche

1. Rotierbarer medizinischer Steckverbinder (10), umfassend:
einen Körper (12);
eine mittige Achse (17), die sich durch den Körper erstreckt;
zumindest ein Greifmerkmal (15), das sich aus dem Körper erstreckt, wobei das zumindest eine Greifmerkmal eine erste im Wesentlichen konvexe Oberfläche (38a) und eine zweite im Wesentlichen konkave Oberfläche (40a) auf einer generell entgegengesetzten Seite davon umfasst, wobei das zumindest eine Greifmerkmal weiter ein erstes Ende (34a), ein zweites Ende (36a), ein distales Segment (42), das nahe dem zweiten Ende positioniert ist und ein proximales Segment (44) umfasst, das näher zum ersten Ende als das distale Segment positioniert ist, wobei sich das proximale Segment in einer Richtung erstreckt, die eine größere radiale Komponente in Bezug auf die mittige Achse als die Richtung des distalen Segments aufweist, wobei sich das distale Segment in einer Richtung erstreckt, die generell zirkumferenziell um die mittige Achse ist;
wobei die im Wesentlichen konvexe Oberfläche im Wesentlichen größere Fingerkontaktfläche als die im Wesentlichen konkave Oberfläche derart bereitstellt, dass der Steckverbinder konfiguriert ist, die Gefahr des zu starken Anziehens zu verringern und die Leichtigkeit des Lösens des Steckverbinders von einem anderen medizinischen Geräts zu erhöhen.

2. Rotierbarer Steckverbinder nach Anspruch 1, weiter umfassend:
einen ersten Hohlraum (14) nahe einem proximalen Ende des Körpers und einen zweiten Hohlraum (16) nahe einem distalen Ende des Körpers, wobei die ersten und zweiten Hohlräume einen Durchgang (11) durch zumindest einen Teil einer Länge des Körpers bilden;
wobei sich das Greifmerkmal asymmetrisch um eine Ebene herum erstreckt, welche die mittige Achse enthält.

3. Rotierbarer Steckverbinder nach Ansprüchen 1 oder 2, wobei das Greifmerkmal einen proximalen Teil (48) umfasst, der nahe der mittigen Achse positioniert ist, wobei sich eine Mittellinie (50) des proximalen Teils primär radial von der mittigen Achse erstreckt und ein distaler Teil (46) weiter von der mittigen Achse als der proximale Teil positioniert ist, wobei sich eine Mittellinie (50) des distalen Teils generell in einem Winkel von der Mittellinie des proximalen Teils erstreckt.

4. Rotierbarer Steckverbinder nach einem beliebigen vorangehenden Anspruch, wobei das Greifmerkmal einen Flansch (30a) mit zumindest einer nach außen gerichteten Oberfläche umfasst, wobei der Flansch asymmetrisch um irgendeine Ebene ist, welche die mittige Achse enthält.

5. Rotierbarer Steckverbinder nach einem beliebigen vorangehenden Anspruch, wobei das Greifmerkmal einen Flansch mit zumindest einer radial nach außen gerichteten und zumindest einer radial nach innen gerichteten Oberfläche umfasst.

6. Rotierbarer Steckverbinder nach einem beliebigen vorangehenden Anspruch, wobei das Greifmerkmal generell frei ist, um sich im Wesentlichen relativ zum Körper und zur mittigen Achse zu biegen.

7. Rotierbarer Steckverbinder nach einem beliebigen vorangehenden Anspruch, wobei das Greifmerkmal generell inhibiert ist, sich in einer Richtung zu biegen und frei ist, sich in einer anderen, entgegengesetzten Richtung zu bewegen.

8. Rotierbarer Steckverbinder nach einem beliebigen vorangehenden Anspruch, wobei das Greifmerkmal im Wesentlichen um den Körper gebogen wird, wenn Druck auf zumindest eine Oberfläche des Greifmerkmals angewandt wird.

9. Rotierbarer Steckverbinder nach einem beliebigen vorangehenden Anspruch, wobei das Greifmerkmal konfiguriert ist, im Wesentlichen um den Körper zu kollabieren, wenn Druck auf zumindest eine Oberfläche des Greifmerkmals angewandt wird.

10. Rotierbarer Steckverbinder nach einem beliebigen vorangehenden Anspruch, der weiter Gewinde (26) zum Anschließen des Steckverbindergeräts an zumindest ein weiteres Gerät umfasst.

11. Rotierbarer Steckverbinder nach einem beliebigen vorangehenden Anspruch, der weiter zumindest eine konische Oberfläche zur Verheiratung des Steckverbindergeräts mit einem Gerät umfasst.

12. Rotierbarer Steckverbinder nach einem beliebigen vorangehenden Anspruch, wobei sich das Greifmerkmal axial entlang eines Teils des Steckverbinderkörpers erstreckt.

13. Rotierbarer Steckverbinder nach einem beliebigen vorangehenden Anspruch, der weiter zumindest ein zusätzliches Greifmerkmal aufweist, das sich aus dem Körper erstreckt.

14. Rotierbarer Steckverbinder nach einem beliebigen vorangehenden Anspruch, wobei das Greifmerkmal aus anderem Material als der Körper besteht.

15. Rotierbarer Steckverbinder nach einem beliebigen vorangehenden Anspruch, wobei das Greifmerkmal ein kurvenförmiges Querschnittsprofil aufweist.

16. Rotierbarer Steckverbinder nach Anspruch 1, wobei das zumindest eine Greifmerkmal Oberflächen umfasst, die sich in einer generell spiralförmigen Bahn um die mittige Achse erstrecken.

## Revendications

1. Connecteur rotatif médical (10) comprenant :
un corps (12) ;
un axe central (17) s'étendant à travers le corps ;
au moins un élément de préhension (15) s'étendant à partir du corps, ledit au moins un élément de préhension comprenant une première surface sensiblement convexe (38a) et une seconde surface sensiblement concave (40a) sur son côté généralement opposé, ledit au moins un élément de préhension comprenant une première extrémité (34a), une seconde extrémité (36a), un segment distal (42) positionné à proximité de la seconde extrémité, et un segment proximal (44) positionné plus près de la première extrémité que le segment distal, le segment proximal s'étendant dans une direction qui a un composant radial plus important par rapport à l'axe central que la direction du segment distal, le segment distal s'étendant dans une direction qui est généralement circonférentielle autour de l'axe central ;
dans lequel la surface sensiblement convexe fournit une zone de contact de doigt sensiblement plus importante que la surface sensiblement concave, de sorte que le connecteur est configuré pour diminuer le risque de serrage excessif et augmenter la facilité de desserrage du connecteur d'un autre dispositif médical.

2. Connecteur rotatif selon la revendication 1, comprenant:
une première cavité (14) à proximité d'une extrémité proximale du corps, et une seconde cavité (16) à proximité d'une extrémité distale du corps, les première et seconde cavités formant une voie de passage (11) à travers au moins une partie d'une longueur du corps ;
dans lequel l'élément de préhension s'étend de manière asymétrique autour d'un plan quelconque contenant l'axe central.

3. Connecteur rotatif selon les revendications 1 ou 2, dans lequel l'élément de préhension comprend une partie proximale (48) positionnée à proximité de l'axe central, avec une ligne médiane (50) de la partie proximale qui s'étend principalement de manière radiale à partir de l'axe central, et une partie distale (46) plus éloignée de l'axe central que la partie proximale, avec une ligne médiane (50) de la partie distale qui s'étend généralement selon un angle à partir de la ligne médiane de la partie proximale.

4. Connecteur rotatif selon l'une quelconque des revendications précédentes, dans lequel l'élément de préhension comprend un rebord (30a) avec au moins une surface orientée radialement vers l'extérieur, le rebord étant asymétrique autour d'un plan quelconque contenant l'axe central.

5. Connecteur rotatif selon l'une quelconque des revendications précédentes, dans lequel l'élément de préhension comprend un rebord avec au moins une surface orientée radialement vers l'extérieur et une surface orientée radialement vers l'intérieur.

6. Connecteur rotatif selon l'une quelconque des revendications précédentes, dans lequel l'élément de préhension est généralement libre de fléchir sensiblement par rapport au corps et à l'axe central.

7. Connecteur rotatif selon l'une quelconque des revendications précédentes, dans lequel l'élément de préhension est généralement empêché de fléchir dans une direction et libre de se déplacer dans une autre direction opposée.

8. Connecteur rotatif selon l'une quelconque des revendications précédentes, dans lequel l'élément de préhension est sensiblement fléchi autour du corps lorsque la pression est appliquée sur au moins une surface de l'élément de préhension.

9. Connecteur rotatif selon l'une quelconque des revendications précédentes, dans lequel l'élément de préhension est configuré pour se replier sensiblement autour du corps lorsque la pression est appliquée sur au moins une surface de l'élément de préhension.

10. Connecteur rotatif selon l'une quelconque des revendications précédentes, comprenant en outre des filetages (26) pour raccorder le dispositif formant connecteur à au moins un autre dispositif.

11. Connecteur rotatif selon l'une quelconque des revendications précédentes, comprenant au moins une surface progressivement rétrécie pour coupler le dispositif formant connecteur avec un dispositif.

12. Connecteur rotatif selon l'une quelconque des revendications précédentes, dans lequel l'élément de préhension s'étend de manière axiale le long d'une partie du corps du connecteur.

13. Connecteur rotatif selon l'une quelconque des revendications précédentes, comprenant au moins un élément de préhension supplémentaire s'étendant à partir du corps.

14. Connecteur rotatif selon l'une quelconque des revendications précédentes, dans lequel l'élément de préhension est composé d'un matériau différent de celui du corps.

15. Connecteur rotatif selon l'une quelconque des revendications précédentes, dans lequel l'élément de préhension a un profil transversal généralement incurvé.

16. Connecteur rotatif selon la revendication 1, dans lequel le au moins un élément de préhension comprend des surfaces qui s'étendent sur une trajectoire généralement en spirale autour de l'axe central.
